Europäisches Patentamt

European Patent Office

Office européen des brevets

(11) Publication number: **0 209 976 B1**

# EUROPEAN PATENT SPECIFICATION

(45) Date of publication of patent specification: **20.03.91**    (51) Int. Cl.⁵: **A61M 5/315, A61B 5/14**

(21) Application number: **86304369.1**

(22) Date of filing: **09.06.86**

(54) Syringe locking device and method of attaching a locking device to a syringe.

(30) Priority: **10.06.85 US 743127**
**10.06.85 US 743126**

(43) Date of publication of application:
**28.01.87 Bulletin 87/05**

(45) Publication of the grant of the patent:
**20.03.91 Bulletin 91/12**

(84) Designated Contracting States:
**BE DE FR GB NL**

(56) References cited:
**EP-A- 0 032 039**
**DE-A- 2 810 370**
**GB-A- 4 207**

(73) Proprietor: **SHERWOOD MEDICAL COMPANY**
**1831 Olive Street**
**St. Louis, MO 63103(US)**

(72) Inventor: **Burkholder, Richard Allen**
**155 Timberidge**
**St. Charles Missouri 63303(US)**
Inventor: **Ewalt, Paul Robert**
**7133 Weldon Spring Road**
**St. Charles Missouri 63301(US)**
Inventor: **Gilson, Richard Wayne**
**9858 East Dell Drive**
**Dellwood Missouri 63136(US)**
Inventor: **Walker, Clarence Logan**
**643 Foote Avenue**
**Webster Groves Missouri 63119(US)**
Inventor: **Leigh, Harold Golding**
**12556 Postgrove Drive**
**St. Louis Missouri 63141(US)**

(74) Representative: **Porter, Graham Ronald et al**
**c/o Wyeth Laboratories Huntercombe Lane**
**South**
**Taplow Maidenhead Berkshire SL6 0PH(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to medical syringes and more particularly to syringes incorporating locking devices for setting and holding partial vacuums drawn by the syringe.

There are several devices known for restraining a syringe plunger in an extended position while a vacuum is held in the syringe barrel in front of the plunger piston. Such devices might include stepped stops on the plunger such as are shown in U.S. Patents 3,882,849 and 3,938,505 and German Offenlegungsschrift 2810370. These stepped stop devices, however, are not capable of a continuous range of partial vacuums but instead provide only discrete vacuum levels corresponding to the steps on the plunger. Devices which can provide continuous ranges of partial vacuums are known, but these typically require a separate moving part such as the cam shown in U.S. Patent 4,386,606. Such moving parts may be relatively difficult to manufacture and to use, and may be less reliable.

The present invention provides an improved syringe lock which overcomes the aforementioned disadvantages and which may be fitted to a standard syringe without prior structural modification.

According to the present invention there is provided a syringe comprising a syringe barrel having a bore therein and distal and proximal ends, a syringe plunger locking device comprising a collar fixed to said proximal end of said barrel, said locking device including locking means projecting into said bore, and a syringe plunger axially and rotatably moveable within said bore, said plunger having a longitudinal axis and radial projecting means fixedly projecting radially of said longitudinal axis, characterised in that said collar has attachment means for preventing relative axial movement and relative rotation of said barrel and said locking means is constructed of a first material and said projecting means is constructed of a second material, one of said materials being softer than the other of said materials, said plunger being rotatable from a first position wherein said plunger is axially movable in said bore to a second position wherein said projecting means contacts said locking means and upon said contact of said projection means with said locking means said one of said materials is deformed by the other of said materials wherein said plunger is axially locked to said locking device and in said bore. The locking means preferably engages the plunger on relative rotation of the plunger and barrel and is released by reverse rotation.

The locking device may be easily and quickly installed on a conventional syringe and is, in one preferred embodiment made as a single plastics moulding. The device has no moving parts and is securely retained on the syringe barrel. The device can lock the plunger in any position with respect to the barrel to give a continuous range of partial vacuums.

The locking device may also include stop means to limit relative locking rotation of the syringe barrel and plunger. The stop means prevent excessive rotation which may leave the parts in an unlocked condition, and also provide a fixed stop against which an unskilled user can twist the parts without fear of damage or an insecure lock.

The chordal edge thickness of the flange may be thinner than the overall thickness of the collar and the thickness of the flange may increase gradually from the chordal edge.

The collar has two opposed flanges which may be substantially parallel.

The stop means may extend the full depth of the collar at the chordal edge.

In a preferred embodiment the stop means are provided on each of two opposed parallel flanges, the stop means being diametrically opposed on either side of a diametrical line perpendicular to the chordal edge of said flanges.

The attachment means may comprise opposed hooks or ears for engagement with the finger support of the barrel. The hooks comprise axially extending projections which are radially inwardly directed at their tips so as to hook around the usual barrel finger support.

The plunger piston is necessarily of slightly larger diameter than the bore diameter of the syringe body in order to give an effective seal. Care needs to be taken on insertion of the plunger through the locking device to avoid damage to the piston and possible contamination of the syringe bore. In an alternative embodiment the locking device is of interlocking parts which fit around the plunger on the mouth of the barrel. Such a two part device can be installed without removing the plunger from the barrel. The locking device may comprise two parts having interengaging portions which 'snap' together to form a unitary assembly. In a preferred embodiment the parts are identical, each having locking means and co-operating male and female members which interlock to form an assembly.

Preferably the syringe plunger is of cruciform cross-section and at least one of the plunger ribs engages with said locking means to jam the plunger against axial movement relative to the barrel.

The locking means is of a first material and the plunger is of a second material, one of the materials being harder than the other whereby engagement of the locking means and plunger in use causes the harder of said materials to deform or bite into the softer of said materials to lock the plunger against relative axial movement. In a preferred embodiment the locking device is moulded from a relatively hard plastics material such as the glass filled polyester sold by Du Pont under the trade name RYNITE and the syringe plunger is of a relatively softer plastics material such as polypropylene.

Other features of the invention will be apparent from the following description of two preferred embodiments shown by way of example only in the accompanying drawings in which:-

Figure 1 is an exploded perspective drawing of aspirating biopsy needle apparatus with a locking device according to the present invention;

Figure 2 is an elevation of a stylet for use with the apparatus of Figure 1;

Figure 3 is a plan view of apparatus of the present invention including the stylet of Figure 2 penetrating a body at the site of tissue from which a specimen is to be taken;

Figure 4 is a plan view of the assembled apparatus of Figure 1 immediately prior to taking a specimen;

Figure 5 is a plan view of the assembled apparatus of Figure 1 immediately after taking a specimen;

Figure 6 is a section, taken along line 6-6 of Figure 4, showing one embodiment of the locking device according to the present invention;

Figure 7 is a section, taken along line 7-7 of Figure 5, showing the plunger of the syringe locked against movement with respect to the barrel by the locking device of Figure 6;

Figure 8 is a view of the underside of the locking device shown in Figure 6;

Figure 9 is a section taken along line 9-9 of Figure 8;

Figure 10 is a section, taken along line 10-10 of Figure 8;

Figure 11 is an enlarged view of a portion of Figure 10;

Figure 12 corresponds to Figure 6 and shows an alternative embodiment of the locking device;

Figure 13 corresponds to Figure 12 but shows the syringe in the locked condition;

Figure 14 is a plan view of the alternative embodiment with the two identical locking device halves separated;

Figure 15 is a view of the underside of the alternative embodiment in the assembled condition;

Figure 16 is a section taken along line 16-16 of Figure 14;

Figure 17 is a partial section on an enlarged scale, taken along line 17-17 of Figure 14 and showing the syringe in dotted outline;

Figure 18 is a section taken along line 18-18 of Figure 15 and also showing the syringe body in dotted outline; and

Figure 19 illustrates the assembly of the alternative embodiment.

Similar reference characters indicate similar parts throughout the several views of the drawings.

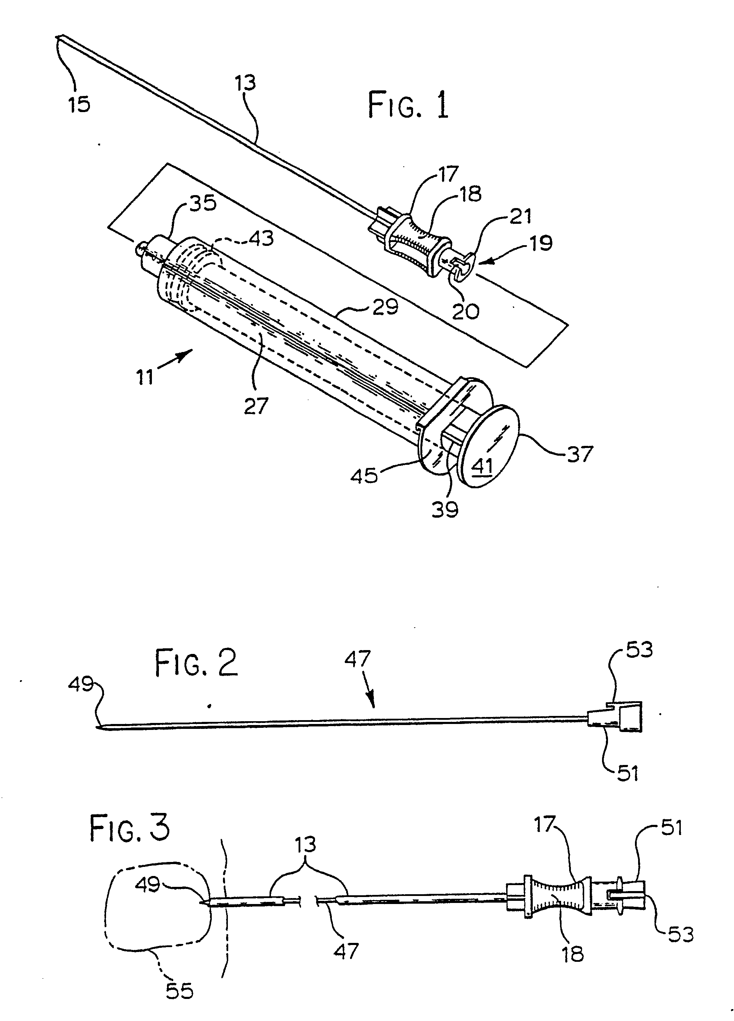

Turning now to the drawings, there is shown (Figure 1) soft tissue aspiration biopsy apparatus 11 which illustrates the use of the locking device of the present invention. Apparatus 11 includes a biopsy needle 13 with a hollow core for receiving a tissue specimen or sample. The needle has a cutting tip 15 at its distal end and is secured to a hub 17 at its opposite, proximal end. Hub 17 has a finger-grip 18 by which it can be grasped for insertion of needle 13 into a body. Hub 17 also has a hollow core, indicated at 19, and a keyway 20 extending distally from its proximal end. Hub 17 terminates at its proximal end in a female luer-lock fitting 21 which is engageable with a corresponding male luer-lock fitting 35 disposed at the distal end of a barrel 27 of a syringe 29. When needle hub 17 is secured by means of the luer-lock fitting to syringe 29, the interior of the needle is in fluid communication with the interior of barrel 27 so that partial vacuums generated inside the barrel are applied to the hollow core of biopsy needle 13.

Syringe 29 further includes a plunger 37 disposed in the syringe barrel, which plunger is reciprocable with respect to the barrel by means of a plunger shaft 39 and a handle 41 attached thereto. Shaft 39 consists of four ribs, each of which extends out from the axis of the shaft at right angles to its neighbours and terminates at its distal end with a plunger tip 43 which slidingly engages the inner wall of barrel 27 to form a seal. A locking device or collar 45 of the present invention is disposed at the proximal end of barrel 27, is exterior thereto, and constitutes means for locking the plunger shaft with respect to the barrel to immobilize the plunger at any desired position with respect to the barrel, whereby any of a desired continuous range of partial vacuums may be applied to the core of biopsy needle 13. Locking device 45 is fixed against rotation with respect to the barrel and may be engaged by the plunger shaft as discussed below to lock shaft 39 against longitudinal movement with respect to barrel 29.

Apparatus 11 also includes a stylet 47 (Figure 2) having a conical pointed tip 49 at one end and a hub 51 at the other. Hub 51 includes a key 53 sized to fit in keyway 20 of a biopsy needle hub 17 to prevent relative rotation of the biopsy needle and the stylet during insertion of the biopsy needle (as shown in Figure 3) into a body from which a specimen is to be taken. Stylet 47 is sized to extend through the hollow cores of biopsy needle 13 and hub 17 so that the pointed tip of the stylet extends past cutting tip 15 of the needle when the stylet is fully inserted into needle 13. This prevents coring as the needle is advanced into the body and the concomitant contamination of the specimen with extraneous tissue. The outside diameter of stylet 47 is, for example and not by way of limitation, approximately 5/10,000 of an inch (0.01 mm) smaller than the needle diameter of needle 13 to permit the stylet to be easily inserted into needle 13 and removed therefrom while still preventing coring. Stylet 47 is completely removable from needle 13 so that once the needle and stylet have been inserted to a desired location in the body as shown in Figure 3 adjacent the tissue 55 to be sampled, the stylet may be removed from the biopsy needle. Syringe 29 with needle hub 23 attached thereto may be prefilled with a small amount of saline solution at this or any suitable earlier time and then attached to the hub of biopsy needle 13. Once hub 17 is secured to the syringe, the interior of needle 13 is in fluid communication with the interior of barrel 27.

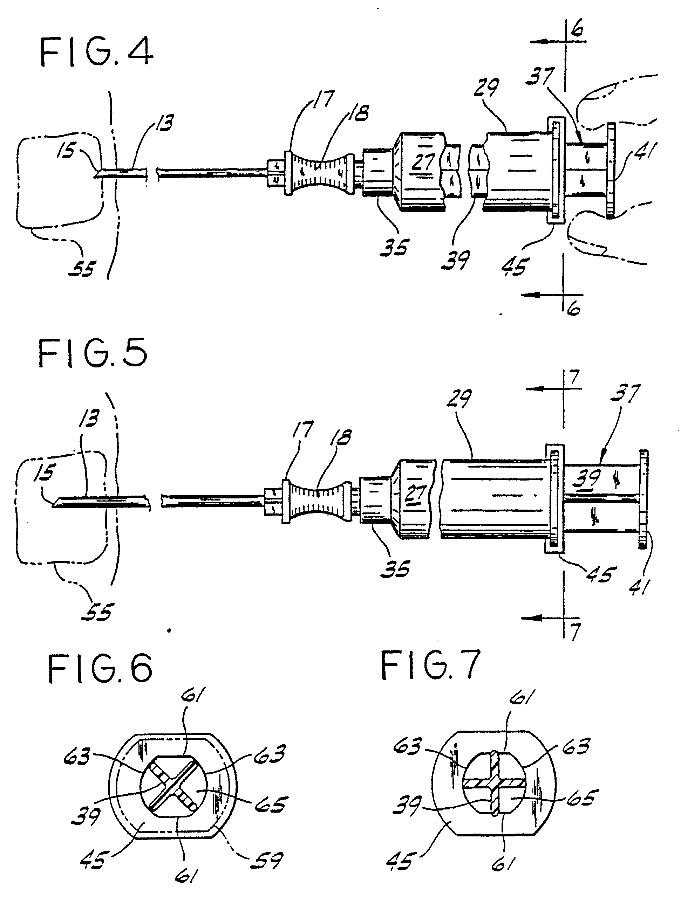

To draw a partial vacuum in needle 13, the user thereupon withdraws the plunger 37 with respect to the barrel 27 from the position shown in Figure 4 to any desired position such as that shown in Figure 5. When the desired vacuum is attained, the user rotates the plunger shaft approximately 45° with respect to the barrel to lock it in place (as is explained below) and pushes the assembly into the tissue to be sampled as shown in Figure 5. A needle 13 is forced into the tissue, tip 15 cuts off a specimen and the vacuum draws the specimen into the needle. Upon removal of the needle from the body, the specimen can be expelled into a suitable receptacle by rotating the plunger shaft to unlock the plunger with respect to the barrel and then pushing the plunger toward the distal end of the syringe to expel the specimen.

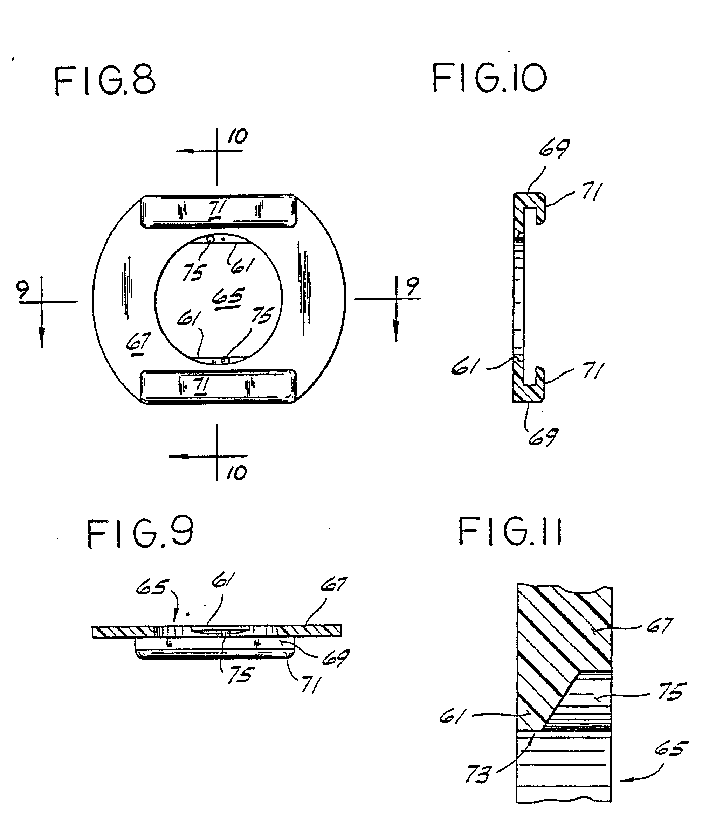

Locking device 45 is a single moulded piece of phenylene oxide or some other suitable hard resin. Syringe barrel 27 includes a finger grip 59 over which locking device 45 is slid during the manufacturing process. More specifically, finger grip 59 has two flat edges and two rounded edges. Device 45 has generally the same outline, but is larger, and has a pair of ears 69 (see Figures 9 and 10) which extend over and behind the flat edges of the finger grip to hold the device against rotation with respect to the barrel once it is placed over the finger grip. Device 45 is dimensioned to provide a friction fit between itself and the finger grip although the tightness of the fit is not critical to the present invention.

Device 45 has a pair of generally flat camming surfaces 61 (Figure 6), which in the embodiment shown are parallel to the flat edges of device 45 but which may assume other orientations, and a pair of curved surfaces 63 therebetween which define an opening 65 through which the shaft of plunger 37 passes. When the plunger shaft has the orientation shown in Figure 6, the plunger shaft is easily movable with respect to locking device 45 along its longitudinal axis. However, when shaft 39 is rotated to the position shown in Figure 7 it is held against further longitudinal movement because the distance between flat surfaces 61 is less than the transverse width of the plunger shaft measured along the ribs. The plunger shaft may simply deform or jam against locking device or the surfaces 61 may score or cut into the shaft to give a more positive engagement. In the preferred embodiment polypropylene is used for the plunger shaft and a relatively harder material such as the glass-filled polyester sold by Du Pont under the trade name RYNITE is used for the locking device. Alternatively the material of the locking device may be softer than that of the plunger. In either case, the resulting tight friction fit prevents relative motion between the barrel and the plunger once the plunger is locked in place, thus freeing the user's hands. RYNITE has a hardness of R120 on the Rockwell scale.

Device 45 (shown in more detail in Figures 8 to 11) has a generally flat body 67 in which opening 65 is centered. Generally flat camming surfaces 61 are seen from Figures 8 and 9 to be ridges which extend chordally across opening 65 and which are substantially parallel to each other. A pair of ears 69 extend out perpendicularly from the plane of flat body 67 toward the distal end of the syringe, each ear 69 terminating in an inwardly extending lip 71. Ears 69 and lips 71 are dimensioned to slide over finger grip 59 of the syringe and thus constitute means adapted to fit on the barrel 27 for holding the locking device against rotation.

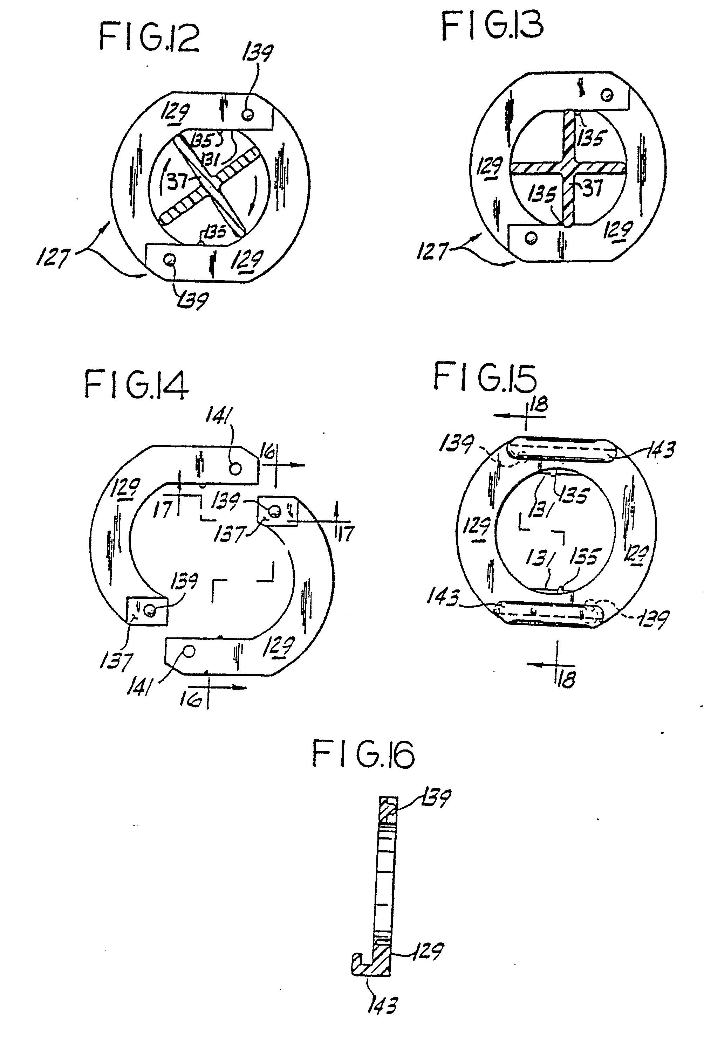

Ridges 61 are not as thick as flat body 67 (see Figures 9 and 11) but are tapered away from the central opening for strength, ease of manufacture, and to provide a relatively thin cutting surface 73 for cutting into or suitably depressing the rib of plunger shaft 39 which is brought into rotation therewith. To ensure that the plunger shaft upon rotation to the locking position shown in Figure 7 does not rotate past the locking position, and hence free the plunger inadvertently, a post or stop 75 is provided on each ridge 61. Posts 75 are disposed on opposite sides of a centre line of opening 65 which extends perpendicular to both ridges to allow the plunger shaft to be rotated to the position shown in Figure 7 and are thicker than the ridges (that is they extend distally out from the ridges). As shown in Figure 9, post 75 extends the full depth of flat body 67 and thus provides a positive stop for plunger shaft 39.

An alternative embodiment of the invention is shown in Figures 12 to 19.

The locking device 127 (see Figure 12) consists of two identical device halves 129 which interlock with each other to form device 127. Although the halves are shown as identical, it should be appreciated that non-identical mating or interlocking parts could also be used. Each device half has a generally semi-circular inner perimeter and a ridge 131 extending chordally across the inner perimeter constituting means for engaging the ribs of plunger 37 when locking is desired. When assembled, the two device halves define the generally circular inner opening shown in Figures 12 and 13, which opening has a diameter at least as large as the width of plunger 37. The assembled device has substantially the same appearance and function as the embodiment of Figures 1 to 11. Clockwise rotation of plunger 37 will lock the plunger against axial movement of the syringe barrel.

A stop 135 is provided on each device half to ensure that the plunger shaft is not accidentally rotated past the position shown in Figure 13 to another unlocked position. The precise size, placement, and shape of stop 135 is not critical. It is merely desired that it provide a positive stopping action in the locked position and that it be easily mouldable as an integral part of locking device half 129.

Although the body of locking device 127 is generally flat with a flat upper surface, each device half has a mating recess 137 (see Figure 14) in which is disposed a post or male member 139 which extends perpendicularly out from the surface of the recess but which terminates before the flat upper surface of the device itself. The opposite end of each device half 129 includes a female member or opening 141 sized to receive post 139, post 139 terminating within opening 141. Of course, methods of engagement other than a post and hole could be used to lock together the two device halves.

Each locking device half 129 (see Figures 15 and 16) includes an ear 143 similar to that shown in the embodiment of Figures 1 to 11. In addition to showing the exact shape of ear 143, Figure 16 also more clearly reveals that post 139 terminates at or below the upper surface of the locking device, so that the flat upper surface of the assembled device is not interrupted.

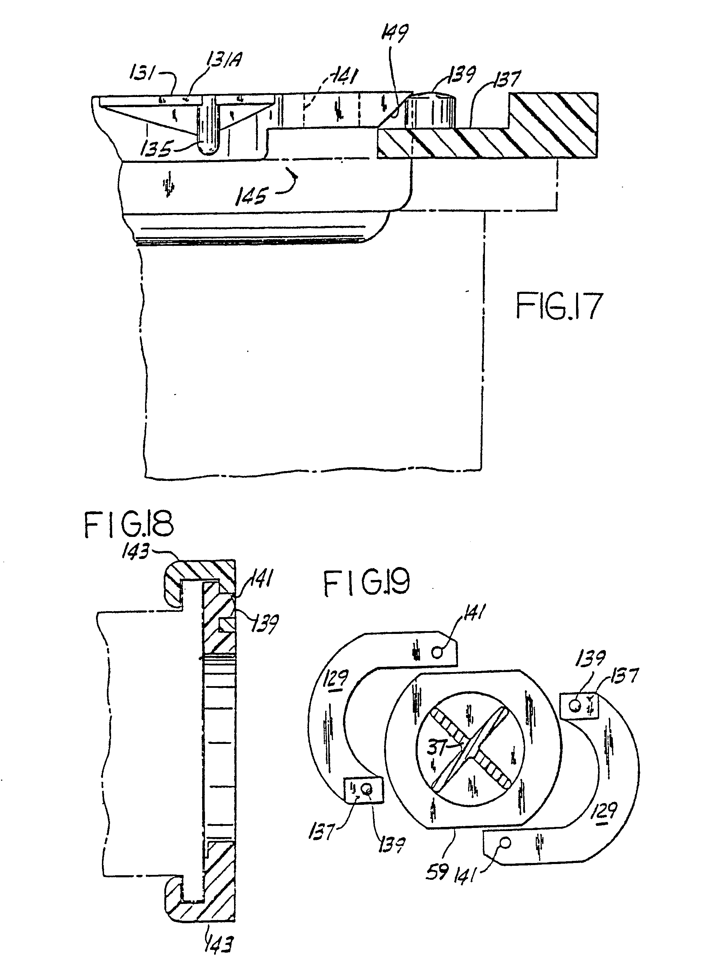

Ridge 131 (see Figure 17) of each locking device half has a relatively narrow upper surface 131A suitable for cutting into or indenting the plunger ribs. Ridge 131 widens as it moves away from the central opening. Stop 135 is moulded integrally with the ridge but may extend out therefrom for the reasons set forth above. The distal side of device half 129 is notched out as indicated at 145 in the vicinity of opening 141 to provide room for the mating surface 147 of the other device half without increasing the overall thickness of the device half. The body of the device half includes a ramp 149 leading to notch 145 for reasons which will become apparent. The locking device is preferably assembled after the plunger and piston is inserted into the syringe barrel. More specifically, both device halves are placed generally as shown in Figure 19, with the ear 143 of each device half disposed in position about its corresponding finger grip and the post of each abutting the corresponding ramp 149 of the other device half. Then, pressure applied to the outside of each device half to force them together causes each ramp 149 to ride up over corresponding post 139 until the posts snap into openings 141. In this position (see Figure 18) the device halves hold each other in place at the proximal end of the syringe barrel with the ears of the locking device secured about the finger grip of the syringe barrel.

Other embodiments of the invention will be apparent from the foregoing description. For example the syringe plunger may have greater or fewer longitudinal ribs and the locking device may act on fewer or greater ribs. Various changes, adaptations and modifications are possible without departing from the scope of the invention.

## Claims

1. A syringe comprising a syringe barrel (27) having a bore therein and distal and proximal ends, a syringe plunger locking device comprising a collar (45) fixed to said proximal end of said barrel, said locking device (45) including locking means projecting into said bore, and a syringe plunger (37) axially and rotatably moveable within said bore, said plunger (37) having a longitudinal axis and radial projecting means fixedly projecting radially of said longitudinal axis, characterised in that said collar has attaohment means for preventing relative axial movement and relative rotation of said barrel (29) and said locking means is constructed of a first material and said projecting means is constructed of a second material, one of said materials being softer than the other of said materials, said plunger (37) being rotatable from a first position wherein said plunger (37) is axially movable in said bore to a second position wherein said projecting means contacts said locking means and upon said contact of said projection means with said locking means said one of said materials is deformed by the other of said materials wherein said plunger (37) is axially locked to said locking device and in said bore.

2. A syringe according to Claim 1, wherein said collar (45) has a generally ciroular aperture and

said locking means on said collar (45) comprises two inwardly directed opposed flanges (61) extending chordally across said aperture and the chordal edge thickness of each flange (61) are thinner than said collar (45).

3. A syringe according to Claim 1 or 2, wherein said locking means further includes a stop means to limit relative rotation of said plunger (37).

4. A syringe according to Claim 3, wherein said stop means comprises abutments (75) on the chordal edges of said flanges (61).

5. A syringe according to any one of Claims 2-4, wherein the thickness of said flanges (61) increase gradually from said chordal edge.

6. A syringe according to Claim 3 or Claim 4, wherein said stop means is of greater thickness than said chordal edge.

7. A syringe according to any one of Claims 2 - 6, wherein the respective chordal edges of said flanges (61) are substantially parallel.

8. A syringe according to Claim 4, wherein said stop means are diametrically opposed and on either side of a diametrical line perpendicular to the chordal edges of said flanges (61).

9. A syringe according to any one of Claim 1 - 8, wherein said attachment means comprises hooks (69) for engagement, in use, with the finger support of said barrel (29).

10. A syringe according to Claim 9, wherein two opposed hooks (69) are provided, each hook comprising a projection extending axially of said collar and having a radially inwardly directed member (71) at the end thereof.

11. A syringe according to any one of Claims 1 - 10, wherein the collar is a single piece plastic moulding.

12. A syringe according to any one of Claims 1 - 10, wherein said collar comprises interlocking parts.

13. A syringe according to Claim 12, wherein two parts are provided, each having locking means.

14. A syringe according to Claim 12 or 13, wherein said parts are identical.

15. A syringe according to Claim 13 or 14, wherein each of said parts has a respective projection (139) and recess (141) for interlocking engagement.

16. A syringe according to any one of the preceding Claims, wherein said syringe has a finger support at a proximal end of the barrel and said locking device is mounted on said finger support.

17. A syringe according to Claim 16, wherein the projecting means of said plunger include ribs extending radially from the longtitudinal axis thereof, said ribs being engageable with said locking means on relative rotation of said plunger and barrel.

18. A syringe according to Claim 17, wherein four ribs are provided in cruciform section.

19. A syringe according to any one of the preceding Claims, wherein the locking means is the harder material.

20. A method of assembling a syringe as claimed in any one of Claims 12 to 15 and comprising the steps of:

inserting the plunger into the syringe barrel;

positioning the parts of the locking device around the finger support of the barrel; and

urging the parts of the locking device toward one another such that the parts interlock to form the collar for the proximal end of the barrel.

**Revendications**

1. Seringue (27) comprenant un fût présentant une lumière et des extrémités distale et proximale, un dispositif de blocage de piston de seringue comprenant une bague (45) fixée à l'extrémité proximale du fût, ce dispositif de blocage (45) comportant des moyens de blocage faisant saillie dans la lumière et un piston de seringue (37) pouvant exécuter des mouvements de déplacement axial et de rotation dans la lumière, le piston (37) présentant un axe longitudinal et des saillies radiales fixes s'étendant radialement sur l'axe longitudinal, caractérisée en ce que la bague comporte des moyens de fixation servant à empêcher tout déplacement axial relatif et toute rotation relative du fût (29), lesdits moyens de blocage étant faits d'une première matière et lesdites saillies étant faites d'une seconde matière, l'une de ces matières étant plus souple que l'autre, le piston (37) pouvant tourner à partir d'une première position, dans laquelle il peut se déplacer axialement dans la lumière vers une seconde position, dans laquelle les saillies sont en contact avec les moyens de blocage et où suite audit contact des saillies avec les moyens de blocage, l'une des matières est déformée par l'autre et le piston (37) est bloqué dans le sens axial contre le dispositif de blocage et dans la lumière.

2. Seringue suivant la revendication 1, dans laquelle la bague (45) présente une ouverture dans l'ensemble circulaire et les moyens de blocage sur la bague (45) comprennent deux joues (61) opposées orientées vers l'intérieur qui s'étendent en corde dans l'ouverture, l'épaisseur de chaque joue (61), au niveau du bord formant corde, étant inférieure à celle de la bague (45).

3. Seringue suivant la revendication 1 ou 2, dans laquelle les moyens de blocage comprennent, en outre, un moyen d'arrêt servant à limiter la rotation relative du piston (37).

4. Seringue suivant la revendication 3, dans laquelle les moyens d'arrêt comprennent des butées (75) sur les bords formant corde des joues (61).

5. Seringue suivant l'une quelconque des revendications 2 à 4, dans laquelle l'épaisseur des joues (61) augmente graduellement à partir du bord formant corde.

6. Seringue suivant la revendication 3 ou 4, dans laquelle les moyens d'arrêt ont une épaisseur supérieure à celle du bord formant corde.

7. Seringue suivant l'une quelconque des revendications 2 à 6, dans laquelle les bords formant corde respectifs des joues (61) sont en substance parallèles.

8. Seringue suivant la revendication 4, dans laquelle les moyens d'arrêt sont diamétralement opposés et sont situés de part et d'autre d'une ligne diamétrale qui est perpendiculaire aux bords formant corde des joues (61).

9. Seringue suivant l'une quelconque des revendications 1 à 8, dans laquelle les moyens de fixation comprennent des crochets (69) qui s'engagent, en service, avec le repose-doigt du fût (29).

10. Seringue suivant la revendication 9, dans laquelle deux crochets opposés (69) sont prévus, chaque crochet comprenant une saillie qui s'étend dans le sens axial de la bague et présente, à son extrémité, un organe (71) dirigé radialement vers l'intérieur.

11. Seringue suivant l'une quelconque des revendications 1 à 10, dans laquelle la bague est moulée d'une seule pièce en matière plastique.

12. Seringue suivant l'une quelconque des revendications 1 à 10, dans laquelle la bague comprend des parties qui s'emboîtent.

13. Seringue suivant la revendication 12, dans laquelle sont prévues deux parties présentant chacune des moyens de blocage.

14. Seringue suivant la revendication 12 ou 13, dans laquelle lesdites parties sont identiques.

15. Seringue suivant la revendication 13 ou 14, dans laquelle chacune desdites parties comporte une saillie (139) et un creux (141) respectifs servant à l'emboîtement.

16. Seringue suivant l'une quelconque des revendications précédentes, dans laquelle un repose-doigt est prévu à l'extrémité proximale du fût et le dispositif de blocage est monté sur ce repose-doigt.

17. Seringue suivant la revendication 16, dans laquelle les saillies du piston comportent des nervures qui s'étendent radialement à partir de son axe longitudinal, ces nervures pouvant s'engager avec les moyens de blocage lors d'une rotation relative du piston par rapport au fût.

18. Seringue suivant la revendication 17, dans laquelle sont prévues quatre nervures de section en forme de croix.

19. Seringue suivant l'une quelconque des revendications précédentes, dans laquelle les moyens de blocage sont constitués de la matière la plus dure.

20. Procédé pour assembler une seringue suivant l'une quelconque des revendications 12 à 15, qui comprend les opérations consistant à :
    insérer le piston dans le fût de seringue;
    positionner les parties constituant le dispositif de blocage autour du repose-doigt du fût, et
    solliciter l'une vers l'autre les parties constituant le dispositif de blocage pour qu'elles s'emboîtent afin de former la bague destinée à l'extrémité proximale du fût.

**Ansprüche**

1. Spritze, umfassend einen Spritzenzylinder (27) mit einer Bohrung darin und distalen und proximalen Enden, einer Feststelleinrichtung für den Spritzenkolben, die einen Kragen (45) aufweist, der am proximalen Ende des Zylinders befestigt ist, wobei die Feststelleinrichtung (45) in die Bohrung ragende Sperrmittel aufweist, und einen Spritzenkolben (37), der axial und drehbar in der Bohrung beweglich ist, wobei der Kolben (37) eine Längsachse und radiale Vorsprünge aufweist, die von der Längsachse in radialer Richtung wegragen, **dadurch gekennzeichnet**, daß der Kragen Befestigungsmittel zum Verhindern von relativen Axialbewegungen und relativen Drehbewegungen des Zylinders (29) aufweist und wobei die Feststellmittel aus einem ersten Material und die Vorsprünge aus einem zweiten Material gefertigt sind, wobei eines der Materialien weicher ist als das andere Material, daß der Kolben (37) aus einer ersten Position, in welcher der Kolben (37) in der Bohrung axial beweglich ist, in eine zweite Position, in welcher die Vorsprünge die Feststellmittel berühren, gedreht werden kann, und wobei aufgrund dieser Kontakte der Vorsprünge mit den Feststellmitteln das eine der Materialien durch das andere Material verformt wird, wobei der Kolben (37) an der Feststelleinrichtung in der Bohrung axial befestigt wird.

2. Spritze nach Anspruch 1, worin der Kragen (45) eine im allgemeinen kreisrunde Öffnung hat und die Sperrmittel am Kragen (45) zwei nach innen gerichtete, gegenüber angeordnete Flansche (61) aufweist, die sich als Kreissehne durch die Öffnung erstrecken und wobei die Dicke der Kreissehnenkante jedes Flansches (61) geringer ist als die des Kragens (45).

3. Spritze nach Anspruch 1 oder Anspruch 2, worin die Festtellmittel ferner ein Haltmittel einschließen, das die relative Rotation des Kolbens (37) limitiert.

4. Spritze nach Anspruch 3, worin die Haltemittel Anschläge (75) an der Kreissehnenkante jedes Flansches (61) umfassen.

5. Spritze nach einem der Ansprüche 2 bis 4, worin die Dicke des Flansches (61) von der Kreissehnenkante allmählich zunimmt.

6. Spritze nach Anspruch 3 oder 4, worin die Haltemittel von größerer Dicke als die Kreissehnenkanten sind.

7. Spritze nach einem der Ansprüche 2 bis 6, worin die jeweiligen Kreissehnenkanten des Flansches (61) im wesentlichen parallel sind.

8. Spritze nach Anspruch 4, worin die Haltemittel diametral gegenüber und an beiden Seiten der diametralen Linie senkrecht zu der Kreissehnenkante der Flansche (61) sind.

9. Spritze nach einem der Ansprüche 1 bis 8, worin die Befestigungsmittel Haken umfassen, die mit dem Halter des Zylinders (29) im Eingriff stehen, an den sich die Finger bei der Benutzung anlegen.

10. Spritze nach Anspruch 9, worin zwei gegenüber angeordnete Haken (69) vorgesehen sind, wobei jeder Haken einen axial zum Kragen verlaufenden Vorsprung und ein radial nach innen gerichtetes Teil (71) am Ende aufweist.

11. Spritze nach einem der Ansprüche 1 bis 10, worin der Kragen ein einstückiges Plastikformteil ist.

12. Spritze nach einem der Ansprüche 1 bis 10, worin der Kragen ineinandergreifende Teile umfaßt.

13. Spritze nach Anspruch 12, worin zwei Einzelteile vorgesehen sind, wobei jedes mit Feststellmitteln ausgestattet ist.

14. Spritze nach Anspruch 12 oder Anspruch 13, worin diese Teile identisch sind.

15. Spritze nach Anspruch 13 oder 14, worin jedes dieser Teile einen Vorsprung (139) bzw. eine Vertiefung (141) zum Ineinandergreifen hat.

16. Spritze nach einem der vorhergehenden Ansprüche, worin die Spritze einen Fingerhalter am proximalen Ende des Zylinders hat und die Feststelleinrichtung an diesem Fingerhalter montiert ist.

17. Spritze nach Anspruch 16, worin die Vorsprünge des Kolbens radial von der Längsachse abstehende Rippen sind, und diese Rippen mit den Feststellmitteln beim gegeneinander Verdrehen des Kolbens und des Zylinders in Eingriff bringbar sind.

18. Spritze nach Anspruch 17, worin 4 Rippen in Kreuzform angeordnet sind.

19. Spritze nach einem der vorhergehenden Ansprüche, worin die Feststellmittel aus dem härteren Material sind.

20. Verfahren zum Zusammenbauen der Spritze nach einem der Ansprüche 12 bis 15 mit folgenden Schritten:
    - Einführen des Kolbens in den Spritzenzylinder;
    - Positionieren der Teile der Feststelleinrichtung um den Fingerhalter des Zylinders herum; und
    - zusammendrücken der Teile der Feststellvorrichtung gegeneinander, so daß die Teile ineinander greifen und den Kragen für das proximale Ende des Zylinders bilden.

FIG. 1

FIG. 2

FIG. 3

FIG.4

FIG.5

FIG.6

FIG.7

# FIG.8

# FIG.10

# FIG.9

# FIG.11

FIG.12

FIG.13

FIG.14

FIG.15

FIG.16

FIG.17

FIG.18

FIG.19